(19) Europäisches Patentamt
European Patent Office
Office européen des brevets

(11) Publication number : 0 482 717 B1

(12) # EUROPEAN PATENT SPECIFICATION

(45) Date of publication of patent specification : **08.06.94 Bulletin 94/23**

(51) Int. Cl.$^5$ : **A61K 31/38**

(21) Application number : **91202738.0**

(22) Date of filing : **23.10.91**

(54) **Use of a benzo[b]thiophene-2-carboximidamide derivative against heart failure.**

(30) Priority : **26.10.90 EP 90311744**

(43) Date of publication of application : **29.04.92 Bulletin 92/18**

(45) Publication of the grant of the patent : **08.06.94 Bulletin 94/23**

(84) Designated Contracting States : **AT BE CH DE DK ES FR GB GR IT LI NL SE**

(56) References cited :
**EP-A- 0 026 593**
**EP-A- 0 158 380**
**EP-A- 0 352 832**
**FR-M- 6 997**
**US-A- 4 705 782**
**BR. J. PHARMACOL., vol. 100, no. 4, August 1990, pages 843-849, Macmillan Press Ltd; D.J. MILLER et al.: "The "calcium sensitising" effects of ORG30029 in saponin- or Triton-skinned rat cardiac muscle"**
**A. GOODMAN-GILMAN et al.: "Pharmacological basis of therapeutics", edition 7, 1985, pages 5,10,28, Macmillan Pub., Cie, New York, US**

(73) Proprietor : **Akzo Nobel N.V.
Velperweg 76
NL-6824 BM Arnhem (NL)**

(72) Inventor : **Logan, Robert Thomas
"Leve" 1 Staik Hill
Lanark, Lanarkshire ML11 7PW (GB)**
Inventor : **Redpath, James
5 Etive Crescent
Bishopbriggs, Glasgow (GB)**
Inventor : **McGarry, George
9 St. David's Drive
Airdrie ML6 9QR (GB)**
Inventor : **Roy, Robert Gibson
63 Kenshaw Avenue
Larkhall, Lanarkshire (GB)**

(74) Representative : **Hermans, Franciscus G.M. et al
P.O. Box 20
NL-5340 BH Oss (NL)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention relates to the use of the benzo[b]thiophene-2-carboximidamide derivative having formula I

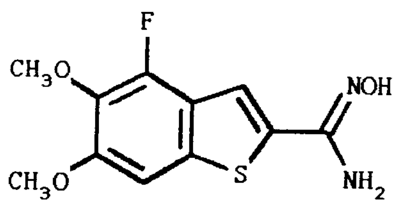

I

or pharmaceutically acceptable salts thereof, for the preparation of a medicament which is suitable for the treatment of heart failure, having high bioavailability without inducing nausea, vomiting and/or restlessness.

The benzo[b]thiophene-2-carboximidamide derivative having formula I was disclosed in European patent application 352,832 as a compound with bronchodilator activity. No activity against the occurence of heart failure was disclosed, nor the advantages of this specific compound with regard to bioavailability and side-effects.

Related compounds with positive inotropic activity have been disclosed in European patent 158,380. During the development of the most promising compound of European patent 158,380, which is compound II

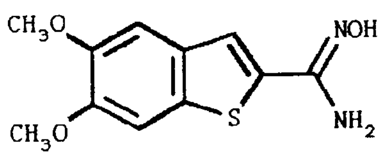

II

it was found that, when administered orally to conscious restrained dogs the compound consistently produces vomiting and restlessness at doses >10 mg/kg. In clinical Phase I studies, nausea and vomiting were also observed after oral administration of compound II to volunteers, thus restricting the maximum tolerated dose, which may be too low to give a sufficient effect on the prevention of heart failure.

It appeared that the 4-chloro substituted derivative III, disclosed in said European patent 158,380

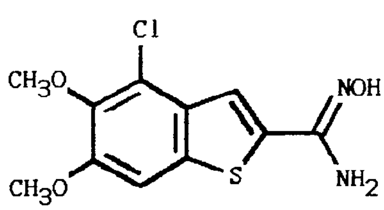

III

did not cause emesis and vomiting, although restlessness was still observed. However, it also appeared that this improvement with regard to compound II could be fully attributed to its reduced bioavailability. Only modest increases of left ventricular contractility (LV dP/dt) were observed in conscious dogs after oral administration of compound III at doses up to, and including, 50 mg/kg. Poor activity was also observed after oral administration (by gavage) of the compound to anaesthetized cats. Substitution with Cl at the 4-position in the benzothiophene nucleus, thus reduces oral activity. On the ground of these results it was assumed that reduction of vomiting, nausea and restlessness could only be obtained at the expense of bioavailability.

Surprisingly, it has now been found that substitution of the aromatic nucleus with fluorine at the 4-position affords a derivative of formula I that is equipotent to the best known compounds, and nevertheless is devoid of side-effects as vomiting, nausea and restlessness.

When administered orally (by gavage) compounds I and II are approximately equipotent at increasing left ventricular contractility (LV dP/dt), but compound I, after oral administration to conscious dogs, is less emetic than compound II. At an oral dose of compound I (25 mg/kg), which produced marked increases in LV dP/dt, no significant vomiting was observed. The difference in emetic threshold between compounds I and II does not appear to be related to poorer bioavailability of compound I. In multiple dose studies in rats, plasma drug

levels were found to be higher after compound I than after an equivalent dose of compound II. On the basis of these results it is anticipated that, in clinical studies, higher doses of compound I cf. compound II may be administered without producing adverse side-effects.

The pharmacological results after oral administration of compounds I (this invention) *vs.* compounds II and III (EP 158,380), are depicted in Table I (cat) and Table II (dog).

Table I (anaesthetized cat)

| Compound | dose (mg/kg) | increase in LV dP/dt (%) |
|---|---|---|
| I | 15 | 60 |
| II | 10 | 65 |
| III | >50 | 15 |

Table II (conscious dog)

| Compound | dose (mg/kg) | increase in LV dP/dt (%) | emesis | restlessness |
|---|---|---|---|---|
| I | 25 | 45 | 0 | 0 |
| II | 25 | 50-100* | + | + |
| III | 25 | 10 | 0 | + |

* Response too variable to measure accurately. Increase partly due to nausea and restlessness.

Compound I of this invention may be prepared according to the methods described in European patent application 352,832 and in European patent 158,380.

A convenient starting product is aldehyde 1, in which R is hydrogen (J. Med. Chem., 1981, 24, 1395), or R is methyl (J. Org. Chem., 1981, 46, 205, and Synthetic Commun., 1985, 15, 61):

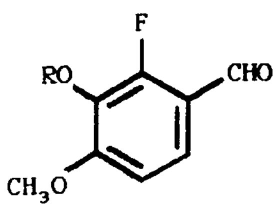

1

R = H or $CH_3$

This aldehyde was converted into the thiol, and cyclisation was achieved by a two stage process, in which chlorine in carbon tetrachloride was added to the thiol in dioxan to give a dimer, which was treated in a separate

reaction with iodine/dioxan, or by a single stage process, in which only iodine/dioxan was used. When R is hydrogen, the hydroxy group was methylated, for instance with iodomethane, after which the esterified carboxylate group was saponified to obtain 4-fluoro-5,6-dimethoxybenzo[b]thiophene-2-carboxylic acid. The resulting reaction steps, i.e. formation of the corresponding 2-nitrile derivative, *via* the corresponding acid chloride and amide, followed by hydroxylamine treatment to obtain compound I, have been described in the previously mentioned European patent and patent application.

Compound 1 thus obtained, may be isolated from the reaction mixture in the form of a pharmaceutically acceptable salt. The pharmaceutically acceptable salts may also be obtained by treating the free base of formula I with an organic or inorganic acid such as HCl, HBr, HI, $H_2SO_4$, $H_3PO_4$, acetic acid, propionic acid, glycolic acid, maleic acid, malonic acid, methanesulphonic acid, fumaric acid, succinic acid, tartaric acid, citric acid, benzoic acid, or ascorbic acid. Preferably compound I is isolated as the methanesulphonic acid salt.

The compounds of the invention may be administered enterally or parenterally, and for humans preferably in a daily dosage of 0,001-10 mg per kg body weight. Mixed with pharmaceutically suitable auxiliaries, e.g. as described in the standard reference, Chase et al., Remington's Pharmaceutical Sciences, the compounds may be compressed into solid dosage units, such as pills, tablets, or be processed into capsules or suppositories.

By means of pharmaceutically suitable liquids the compounds can also be applied as an injection preparation in the form of a solution, suspension, emulsion, or as a spray, e.g. a nasal spray.

The invention is further illustrated by the following examples.

Example 1

A. Sodium metal (2 g), cut into small pieces, was added portionwise to a stirred solution of methanol (30 ml) under an atmosphere of nitrogen. When all sodium had dissolved a hot solution of hydroxylamine hydrochloride (6.1 g) in methanol (35 ml) was added. The resultant suspension was stirred for 1 h, then cooled to room temperature and the sodium chloride precipitate was filtered off and washed with methanol. The filtrate was added to 4-fluoro-5,6-dimethoxybenzo[b]thiophene-2-carbonitrile (7 g) and the mixture was stirred and heated at 40-50 °C. After 1.5 h the mixture was concentrated to 30 ml by vacuum distillation at 30 °C. The suspension was diluted with 70 ml of water, stirred, then filtered and the solid was washed with water and dried at 60 °C under vacuum to give 7.75 g of 4-fluoro-N-hydroxy-5,6-dimethoxybenzo[b]thiophene-2-carboximidamide as a pale yellow solid. M.p. 186-190 °C.

B. Methanesulphonic acid (2 ml) was added to a stirred suspension of 7.75 g of 4-fluoro-N-hydroxy-5,6-dimethoxybenzo[b]thiophene-2-carboximidamide in 20 ml of methanol. The starting material dissolved and the resultant solution was filtered dust-free, then concentrated under reduced pressure. Dust-free diethyl ether (20 ml) was then added to the residual oily crystalline residues and the mixture was stirred until a thick paste had formed. More dust-free diethyl ether (30 ml) was then added, the mixture was stirred again, then the solid was filtered and dried at 65 °C under vacuum to give 10 g of pure 4-fluoro-N-hydroxy-5,6-dimethoxybenzo[b]thiophene-2-carboximidamide methanesulphonate. M.p. 190 °C (dec.).

Example 2

Composition of a tablet (in mg).

| | |
|---|---|
| Cores: | |
| Compound of Example 1 | 20 |
| Maize starch | 12 |
| Hydroxypropylcellulose | 1,5 |
| Magnesium stearate | 0,6 |
| Lactose | to 120 mg |
| Coat: | |
| Hydroxypropylmethylcellulose | 4,375 |
| Polyethylene glycol 400 | 0,875 |
| Titanium dioxide | 0,656 |
| Talc | 1.094 |

White, coated biconvex tablets with a diameter of approx. 7 mm, a radius of convexity of 8 mm and a thickness of approx. 3 mm were obtained.

Example 3

Composition of an injection preparation:

A)

| | | |
|---|---|---|
| Compound of Example 1 | | 10 mg |
| NaCl (to isotone) | approx. | 9 mg |
| Water for injection | | 1 ml |

B)

| | |
|---|---|
| Compound of Example 1 | 10 mg |
| $NaH_2PO_4$ | 3 mg |
| citric acid | 3 mg |
| HCl or NaOH to pH 5 | |
| Water for injection | 1 ml |

Example 4

Composition of a nasal spray:

A)

| | |
|---|---|
| Compound of Example 1 | 30 mg |
| Propylene glycol | 200 mg |
| Methylparahydroxybenzoate | 0,2 mg |
| Propylparahydroxybenzoate | 1 mg |
| $NaH_2PO_4$ | 3 mg |
| Citric acid | 3 mg |
| HCl or NaOH to pH 5 | |
| Water for injection | 1 ml |

B) as A with additional absorption promotor, for instance 10% (w/v) cyclodextrin derivative or 1% (w/v) cholic acid derivative.

## Claims

1. The use of the benzo[b]thiophene-2-carboximidamide derivative having formula I

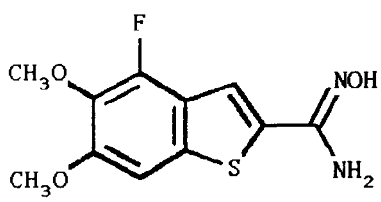

I

or pharmaceutically acceptable salts thereof for the preparation of a medicament which is suitable for the treatment of heart failure, having high bioavailability without inducing nausea, vomiting and/or restlessness.

## Patentansprüche

1. Die Verwendung des Benzo[b]thiophen-2-carboximidamid-Derivats der Formel I

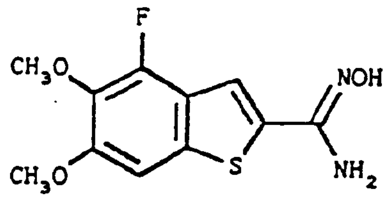

I

oder pharmazeutisch annehmbarer Salze davon, zur Herstellung eines Arzneimittels, das geeignet ist für die Behandlung von Herzversagen, das eine hohe biologische Verfügbarkeit besitzt, ohne Übelkeit, Erbrechen und/oder Ruhelosigkeit zu verursachen.

## Revendications

1. L'utilisation d'un dérivé de dérivé de benzo[b]thiophene-2-carboximidamide représenté par la formule I

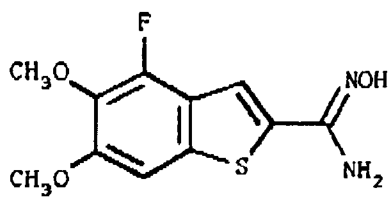

I

ou les sels pharmaceutiquement acceptables de ce dernier, pour la préparation d'un médicament approprié au traitement de l'insuffisance cardiaque, présentant une biodisponibilité élevée sans induire de nausées, de vomissement et/ou d'agitation.